# EUROPEAN PATENT APPLICATION

(11) **EP 0 786 252 A1**
(43) Date of publication of application: **30.07.1997**
(21) Application number: 95932191.0
(22) Date of filing: 20.09.1995
(51) Int. Cl.: A61K 31/40, C07D 487/04

(54) **ANTITUMOR AGENT**

(30) Priority: 30.09.1994 JP 237226/94
(71) Applicant: KYOWA HAKKO KOGYO CO., LTD., Chiyoda-ku, Tokyo 100 (JP)
(72) Inventor: SAITO, Hiromitsu, Kanagawa 214 (JP); AMISHIRO, Nobuyoshi, Sunto-gun Shizuoka 411 (JP); NAGAMURA, Satoru, Yamaguchi 747 (JP); KOBAYASHI, Eiji, Tokyo 157 (JP); OKAMOTO, Akihiko, Shizuoka 410 (JP); GOMI Katsushige, Shizuoka 410-11 (JP); YAMASHITA, Kinya, Shizuoka 411 (JP); SATO, Kiyoshi, Mishima-shi Shizuoka 411 (JP); NAKAKURA, Masashi, Sunto-gun Shizuoka 411 (JP); HAYAKAWA, Eiji, Shizuoka 410-11 (JP)
(74) Representative: Casalonga, Axel
(86) International application number: JP9501893
(87) International publication number: WO9610405

(57) **Abstract**

An anti-tumor agents against drug-resistant tumors, containing, as the active ingredient, a compound represented by the following formula, or its pharmaceutically-acceptable salt: 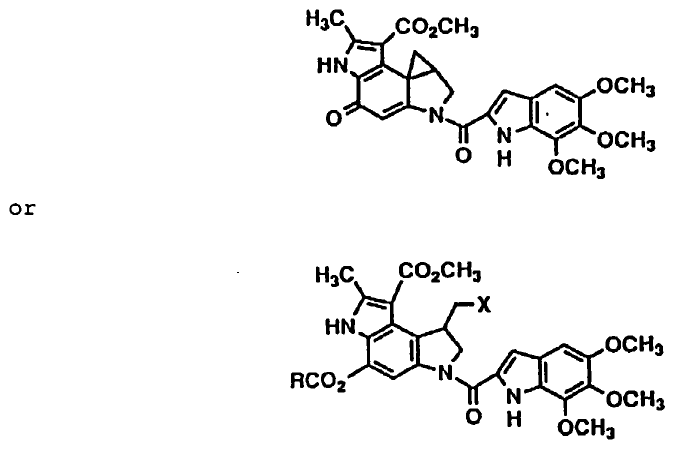 wherein X is Cl or Br;
R is NR¹R² (in which R¹ and R² independently represent hydrogen, or a linear or branched alkyl group with 1 to 4 carbon atoms), or 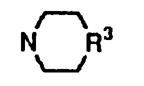 in which R³ is -CH₂- or N-CH₃.

The anti-tumor agent of the present invention exhibits an excellent anti-tumor activity against tumors resistant to clinical anticancer drugs, and is useful as an anti-tumor agent for drug-resistant tumors.

## Description

### Technical Field

The present invention relates to an anti-tumor agent containing a DC-89 derivative. The anti-tumor agent of the present invention exhibits an excellent anti-tumor activity against tumors that are resistant to existing clinical anticancer drugs, and is therefore useful as an anti-tumor agent against drug-resistant tumors.

### Background Art

Japanese Published Unexamined Patent Application Nos. 3-128379 and 5-97583 disclose that DC-89 derivatives and their salts for the anti-tumor agent have an anti-tumor activity and are useful as anti-tumor agents.

### Disclosure of the Invention

The present invention relates to an anti-tumor agent against drug-resistant tumors, containing, as the active ingredient, a compound represented by general formula (I) or a pharmaceutically-acceptable salt thereof: 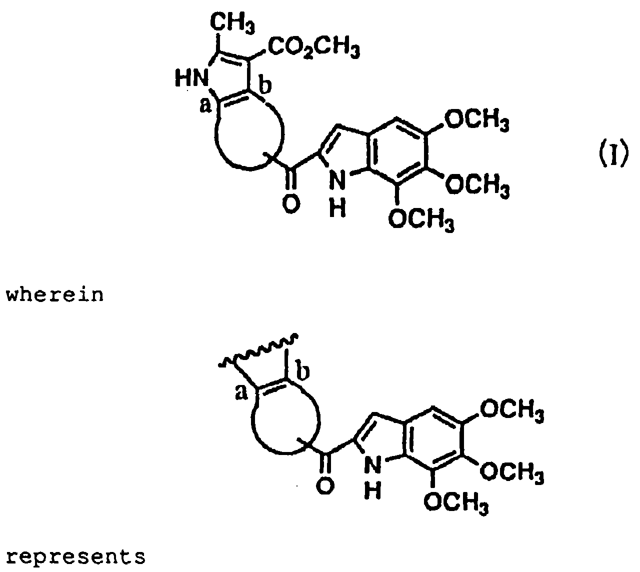 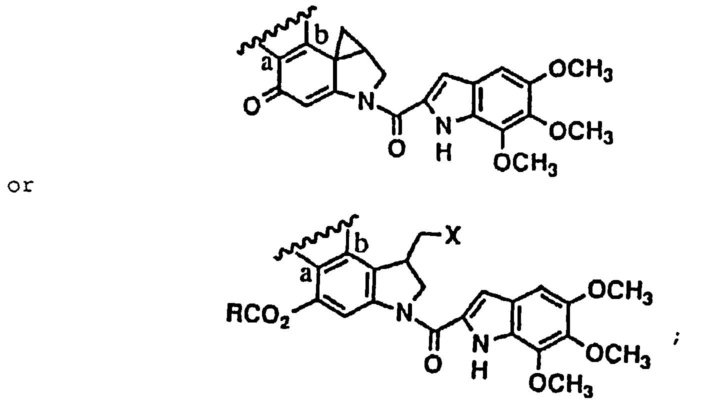 wherein X represents Cl or Br;
R represents NR¹R² (in which R¹ and R² independently represent hydrogen, or a linear or branched alkyl group having 1 to 4 carbon atoms), or represents 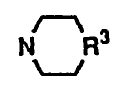 (in which R³ represents -CH₂- or N-CH₃).

The present invention also relates to the use of the compounds of formula (I) or their pharmaceutically-acceptable salts for the production of pharmaceutical compositions effective against drug-resistant tumors.

The compounds of formula (I) are hereinafter referred to as compound (I).

In formula (I), the linear or branched alkyl group having 1 to 4 carbon atoms may include methyl, ethyl, propyl, isopropyl, butyl, isobutyl, sec-butyl and tert-butyl groups.

The pharmaceutically-acceptable salts of compounds (I) may include inorganic acid-added salts such as hydrochlorides, hydrobromides, hydroiodides, nitrates, sulfates and phosphates thereof; and organic acid-added salts such as acetates, benzoates, maleates, fumarates, succinates, tartrates, citrates, oxalates, glyoxalates, aspartates and methanesulfonates thereof.

Compounds (I) can be obtained according to the method described in Japanese Published Unexamined Patent Application No. 3-128379 or 5-97853.

Structures of typical compounds belonging to compound (I) are mentioned below along with the number thereof.

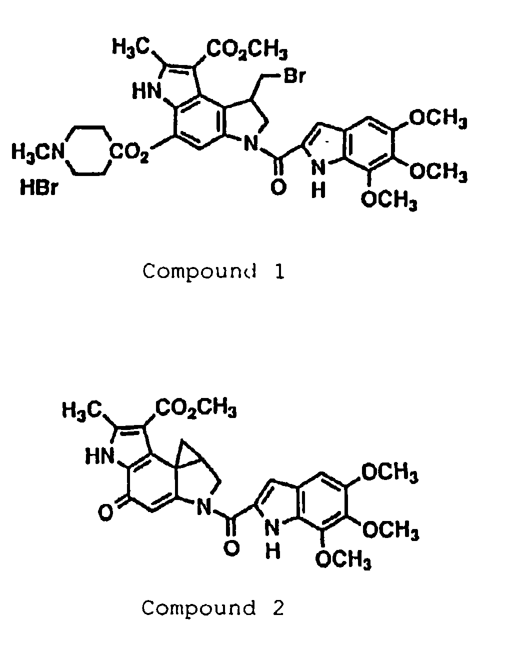

Physicochemical properties of these compounds are shown in Table 1.

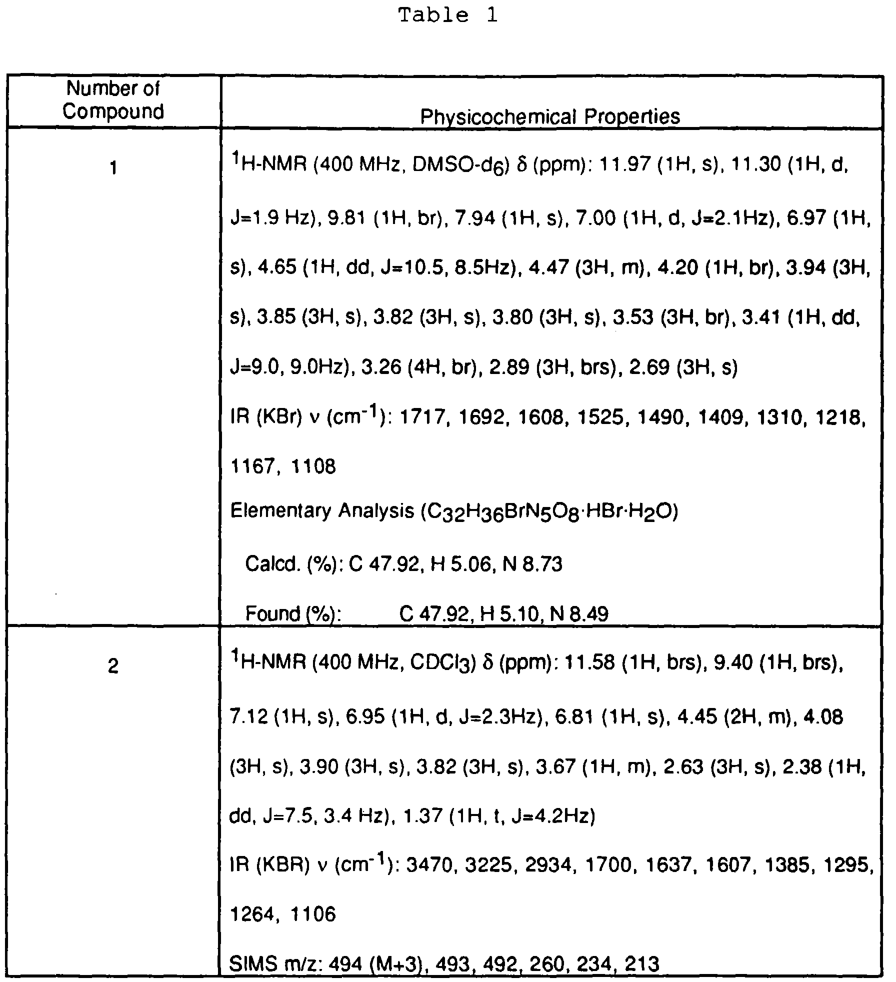

Compounds (I) and their pharmaceutically-acceptable salts have an excellent anti-tumor activity against tumors that are resistant to existing clinical anticancer drugs. The existing clinical anticancer drugs may include adriamycin, cisplatin, cyclophosphamide, mitomycin C, vincristine, and 5-fluorouracil. The tumors resistant to such existing clinical anticancer drugs may include leukemia, gastric cancer, colon cancer, lung cancer, mammary cancer, and uterine cancer.

The following test examples demonstrate the pharmaceutical activities of compound (I).

### Test Example 1

### Antiproliferative Activity against Drug-resistant Tumor Cells

Compound (I) was tested for its antiproliferative activity against lymphocytic leukemia P388 cells, adriamycin-resistant P388 cells (P388/ADM), cisplatin-resistant P388 cells (P388/CDDP), cyclophosphamide-resistant P388 cells (P388/CPM), and mitomycin C-resistant P388 cells (P388/MMC), in the manner described below.

P388 cells were suspended in an RPMI-1640 medium containing 10 % fetal calf serum, 100 units/ml penicillin, 100 µg/ml streptomycin and 20 µM 2-mercaptoethanol at a concentration of 3 x 10⁴ cells/ml, and 0.1 ml of the cell suspension was put into each well of a 96-well microplate. The cells were incubated in a carbon dioxide incubator (5 % carbon dioxide) overnight at 37°C, and 0.05 ml/well of a compound (I) that had been suitably diluted with the medium was added to each well.

The cells were further incubated in the carbon dioxide incubator for 72 hours, then the culture supernatant was removed, and the number of the cells was counted using a cell counter. The number of non-treated cells was compared with that of cells treated with the compound (I), from which was obtained the concentration of the chemical that inhibited 50 % of the cell growth. This is referred to as IC₅₀.

For the others, P388/ADM cells, P388/CDDP cells, P388/CPM cells and P388/MMC cells, IC₅₀ of the compound (I) was obtained in the same manner as above. The data obtained are shown in Table 2.

**Table 2**

| Compound (Compound No.) | IC₅₀ (nM) | | | | |
|---|---|---|---|---|---|
| | P388 | P388/ADM | P388/CDDP | P388/CPM | P388/MMC |
| 1 | 8.17 | 15.6 | 19.8 | 6.34 | 15.3 |
| ADM | 6.62 | 571 | 18.1 | 5.79 | 14.7 |
| CDDP | 75.4 | 88.3 | 4080 | 504 | 1090 |
| MMC | 40.2 | 56.9 | 186 | 94.4 | >300 |
| ADM: Adriamycin CDDP: Cisplatin MMC: Mitomycin C CPM: Cyclophosphamide | | | | | |

As shown in Table 2, the anti-tumor activity of compound (I) against drug-resistant tumors was good.

### Test Example 2

### Acute Toxicity

Compound (I) was intravenously administered to ddY mice (male; body weight, 19 to 21 g). 30 days after the administration, LD₅₀ and LD₁₀ were obtained through probit analysis.

The data obtained are shown in Table 3. As shown in Table 3, LD₅₀ of the compound (I) was 0.81 mg/kg, and LD₁₀ thereof was 0.63 mg/kg.

**Table 3**

| Compound No. | Dose (mg/kg) | Mean Survival Time (days +/- SD) | Death Rate |
|---|---|---|---|
| 1 | 0.48 | No died | 0/20 |
| | 0.57 | 11.6 +/- 3.4 | 2/20 |
| | 0.69 | 11.6 +/- 1.5 | 3/20 |
| | 0.82 | 8.3 +/- 1.8 | 10/20 |
| | 0.99 | 6.8 +/- 1.6 | 17/20 |
| | 1.2 | 5.6 +/- 1.8 | 20/20 |

The anti-tumor agent of the present invention can be used as a pharmaceutical composition, either singly or as combined with at least one or more pharmaceutically-acceptable auxiliary agents. The pharmaceutical composition of the invention can be produced by uniformly mixing compound (I) or its pharmaceutically-acceptable salt in an amount that is effective as the active ingredient, with pharmaceutically-acceptable carriers. It is desirable that the pharmaceutical composition is in the form of a unit dose suitable for oral administration or for administration through injection.

To prepare a pharmaceutical composition for oral administration, any useful pharmaceutically-acceptable carriers can be used. For example, liquid preparations for oral administration such as suspension and syrup can be prepared using water; sugars such as sucrose, sorbitol and fructose; glycols such as polyethylene glycol and propylene glycol; oils such as sesame oil, olive oil and soybean oil; preservatives such as p-hydroxybenzoates; and flavors such as strawberry flavor and peppermint, etc. Powders, pills, capsules and tablets can be prepared using excipients such as lactose, glucose, sucrose, and mannitol; disintegrators such as starch and sodium alginate; lubricants such as magnesium stearate and talc; binders such as polyvinyl alcohol, hydroxypropyl cellulose, and gelatin; surfactants such as fatty acid esters; and plasticizers such as glycerin, etc. To prepare tablets and capsules, solid pharmaceutical carriers are used.

Injectable preparations can be prepared using a carrier such as distilled water, a salt solution, a glucose solution, or a mixture of a salt solution and a glucose solution. The preparations can be prepared in the form of solution, suspension or dispersion according to a conventinal method using a suitable auxiliary.

Compound (I) or its pharmaceutically-acceptable salt may be dissolved in a solution, which is preferably acidic, more preferably at pH of 5 or lower, along with at least one selected from sugars, electrolytes, water-soluble polymers, polyalcohols and surfactants, then filtered through a membrane filter under germ-free conditions, and lyophilized to give lyophilized preparations.

Sugars may include lactose, sucrose, raffinose, and dextran; but preferred is lactose. The concentration of sugar may be 0.005 to 1000 mg/ml, preferably 1 to 500 mg/ml. Electrolytes may be any one that is pharmaceutically acceptable and that may enhance the ionic strength of a solution. Electrolytes may include inorganic acid such as hydrochloric acid, hydrobromic acid, hydroiodic acid, sulfuric acid, carbonic acid, silicic acid, phosphoric acid, and boric acid; organic acids such as citric acid and acetic acid; and their sodium or potassium salts. The concentration of the electrolyte may be 0.001 to 500 mg/ml, preferably 0.01 to 100 mg/ml. The water-soluble polymers may include polyvinyl alcohol, polyvinyl pyrrolidone, polyethylene glycol, and carboxyvinyl polymers. The concentration of the water-soluble polymer may be 1 to 1000 mg/ml, preferably 10 to 500 mg/ml. The polyalcohols may include glycerin and propylene glycol. The concentration of the polyalcohol may be 10 to 1000 mg/ml, preferably 100 to 500 mg/ml. The surfactants may include Tweens and polyoxyethylene hardened castor oil. The concentration of the surfactant may be 0.01 to 5000 mg/ml, preferably 0.1 to 500 mg/ml. The concentration of compound (I) in a lyophilized preparation may be 0.001 to 1000 mg/ml, preferably 0.1 to 10 mg/ml. The lyophilization may be conducted, for example, by freezing the solution at -50°C for 5 hours (for pre-freezing), then at -30°C at 0.05 mbar for 30 hours, and drying it at 0°C at 0.05 mbar for 15 hours (for primary drying) and then at 25°C at 0.05 mbar for 15 hours (for secondary drying), in order.

The lyophilized preparation thus obtained is put into a container and sealed with a rubber stopper or an aluminium cap.

When the lyophilized preparation is used, a solution containing at least one stabilizer selected from electrolytes, water-soluble polymers, polyalcohols and surfactants may be used for dissolving it.

The lyophilized preparation may contain, depending on the object to formulate it, any of pharmaceutically-acceptable antioxidants, antiseptics, buffers, analgesics, solubilizers, dissolution aids, isotonicators, preservatives, stabilizers, vehicles, binders, disintegrators, moisturizers, lubricants, colorants, aromas, flavors, coating agents, suspending agents, emulsifiers, plasticizers, and surfactants. For example, mentioned are antioxidants such as ascorbic acid, vitamin E, butylhydroxytoluene and benzylhydroxytoluene; antiseptics such as parabens and chlorobutanol; buffers such as phosphoric acid and citric acid; analgesics such as benzyl alcohol and lidocaine; vehicles such as crystalline cellulose, hydroxypropyl starch, potato starch and corn starch; binders such as pullulan, polyvinyl alcohol and hydroxypropyl cellulose; disintegrators such as carboxymethyl cellulose and closcarmellose sodium A; and lubricants such as magnesium stearate, talc and hardened oil.

The dose of the composition of the invention varies depending on the age and the condition of a patient, etc. However, Compound (I) of the present invention is administered to mammals including human beings in a dose of 0.001 to 10 mg/kg/day, preferably 0.001 to 0.1 mg/kg/day. For example, the composition may be intravenously administered to a patient once a day (singly or continuously), or intermittently once to three times a week, or once every 2 or 3 weeks. If desired, the composition of the same dose as above may be intra-arterially, intra-abdominally or intrathoracically administered in the same manner as above. If also desired, the composition of the same dose as above may be orally administered.

The anti-tumor agent of the present invention is expected to be effective against chemical-tolerant leukemia, gastric cancer, colon cancer, lung cancer, mammary cancer and uterine cancer in mammals including human beings.

Examples of the present invention are mentioned below.

### Example 1 Lyophilized Preparation

50 mg of citric acid was dissolved in 200 ml of water for injection, to which were added 100 mg of compound 1 and 5000 mg of lactose, and dissolved. The resulting solution was put into 15-ml vials in an amount of 2 ml each, and then lyophilized. After the lyophilization, the vials were restored to be at ordinary pressure in a nitrogen stream, and sealed with a rubber stopper and an aluminium cap. Thus was prepared a lyophilized preparation comprising compound 1.

### Example 2 Lyophilized Preparation

50 mg of citric acid was dissolved in 200 ml of water for injection, to which were added 100 mg of compound 1, 5000 mg of lactose and 100 mg of sodium bromide, and dissolved. The resulting solution was put into 15-ml vials in an amount of 2 ml each, and then lyophilized. After the lyophilization, the vials were restored to be at ordinary pressure in a nitrogen stream, and sealed with a rubber stopper and an aluminium cap. Thus was prepared a lyophilized preparation comprising compound 1.

### Example 3 Lyophilized Preparation

50 mg of citric acid was dissolved in 200 ml of water for injection, to which were added 100 mg of compound 1, 5000 mg of lactose, 100 mg of sodium bromide and 500 mg of Tween 80, and dissolved. The resulting solution was put into 15-ml vials in an amount of 2 ml each, and then lyophilized. After the lyophilization, the vials were restored to be at ordinary pressure in a nitrogen stream, and sealed with a rubber stopper and an aluminium cap. Thus was prepared a lyophilized preparation comprising compound 1.

### Example 4 Lyophilized Preparation

50 mg of citric acid was dissolved in 200 ml of water for injection, to which was added 100 mg of compound 1, and dissolved. The resulting solution was put into 15-ml vials in an amount of 2 ml each, and then lyophilized. After the lyophilization, the vials were restored to be at ordinary pressure in a nitrogen stream, and sealed with a rubber stopper and an aluminium cap. Thus was prepared a lyophilized preparation comprising compound 1.

### Example 5 Tablets

4 g of compound 2, 322.8 g of lactose, and 60 g of potato starch were mixed, to which was added 120 g of 10% hydroxypropyl cellulose aqueous solution. The mixture was kneaded, granulated, dried and then dressed in an ordinary manner to prepare granules to be pelletized. 1.2 g of magnesium stearate was added thereto and mixed, and the resulting mixture was pelletized, using a pelletizer equipped with a pounder having a diameter of 8 mm (RT-15 Model, produced by Kikusui Co.), to obtain tablets (containing 2 mg/tablet of the active ingredient).

| Formulation: | |
|---|---|
| Compound 2 | 2 mg |
| Lactose | 161.4 mg |
| Potato Starch | 30 mg |
| Hydroxypropyl Cellulose | 6 mg |
| Magnesium Stearate | 0.6 mg |
| | 200 mg |

### Example 6 Granules

Granules having the composition mentioned below were produced in an ordinary manner.

2 g of compound 2, 673 g of lactose and 285 g of corn starch were mixed, to which was added 400 g of 10% hydroxypropyl cellulose aqueous solution. The resulting mixture was kneaded, granulated and dried in an ordinary manner to obtain granules (containing 20 g of the active ingredient per 1000 g of granules).

| Formulation: | |
|---|---|
| Compound 2 | 2 mg |
| Lactose | 673 mg |
| Corn Starch | 285 mg |
| Hydroxypropyl Cellulose | 40 mg |
| | 1000 mg |

### Example 7 Capsules

Capsules having the composition mentioned below were produced in an ordinary manner.

20 g of compound 2, 1175 g of avicel and 5 g of magnesium stearate were mixed in an ordinary manner. The resulting mixture was encapsulated, using an Encapsulator (LZ-64 Model, produced by Zanashi Co.), into #4 hard capsules in an amount of 120 mg/capsule to obtain capsules (containing 2 mg/capsule of the active ingredient).

| Formulation: | |
|---|---|
| Compound 2 | 2 mg |
| Avicel | 117.5 mg |
| Magnesium Stearate | 0.5 mg |
| | 120 mg |

### Industrial Applicability

The anti-tumor agent of the present invention exhibits an excellent anti-tumor activity against tumors resistant to clinical anticancer drugs, and is useful as an anti-tumor agent against drug resistant tumors.

## Claims

1. An anti-tumor agent for drug-resistant tumors, containing, as the active ingredient, a compound represented by general formula (I) or a pharmaceutically-acceptable salt thereof: 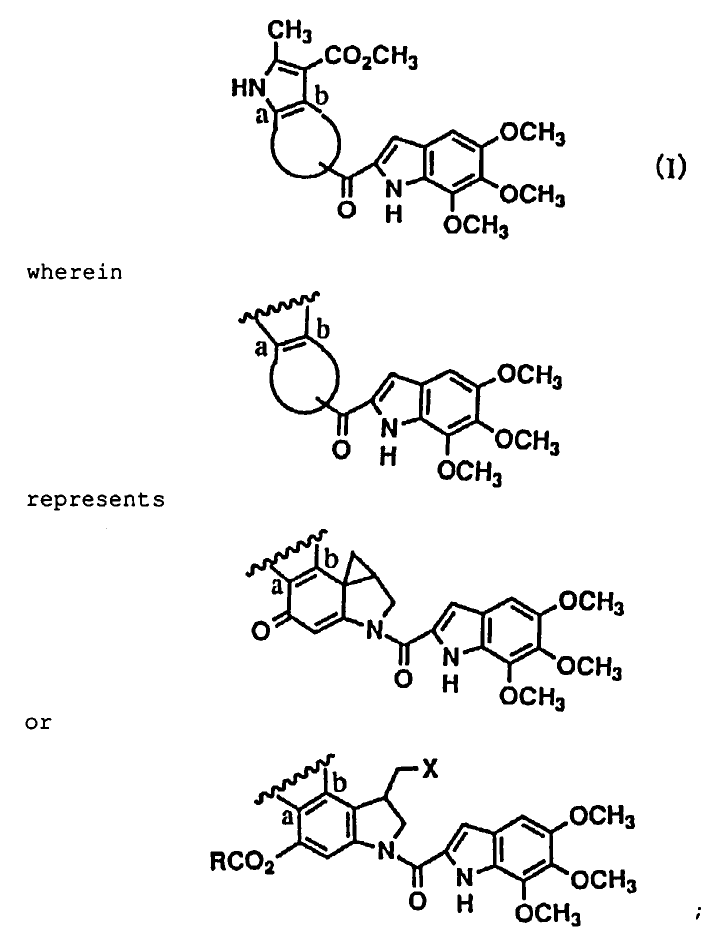 wherein X represents Cl or Br;
R represents NR¹R² (in which R¹ and R² independently represent hydrogen, or a linear or branched alkyl group having 1 to 4 carbon atoms), or represents 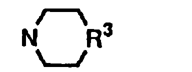 (in which R³ represents -CH₂- or N-CH₃).

2. Use of a compound of formula (I) as set forth in claim 1 or their pharmaceutically-acceptable salts in the production of a pharmaceutical composition effective against drug-resistant tumors.
